# EUROPEAN PATENT APPLICATION

(11) **EP 4 249 670 A2**
(43) Date of publication of application: **27.09.2023**
(21) Application number: 23183851.7
(22) Date of filing: 18.12.2015
(51) Int. Cl.: D06F 58/20, D06F 58/24, D06F 58/10

(54) **CLOTHES TREATMENT APPARATUS**

(30) Priority: 19.12.2014 KR 20140184452
(62) Divisional of application: 19197612.5
(71) Applicant: LG Electronics Inc., Yeongdeungpo-gu Seoul 07336 (KR)
(72) Inventor: PARK, Sunghoo, Seoul (KR); DOH, Youngjin, Seoul (KR); PARK, Hyoungsup, Seoul (KR)
(74) Representative: Vossius & Partner Patentanwälte Rechtsanwälte mbB

(57) **Abstract**

A clothes treatment apparatus is disclosed. The clothes treatment apparatus includes a cabinet partitioned into a treatment chamber for allowing clothes to be hung therein, a cycle chamber for allowing machinery to be installed therein, and a tank installation space for allowing a removable tank comprising a water supply and a drainage tank to be installed therein, a door for opening and closing the cabinet, a steam unit disposed in the cycle chamber for supplying steam into the treatment chamber, a heat pump unit disposed in the cycle chamber for circulating air in the treatment chamber and conditioning the air that is circulated, a water supply tank disposed in the tank installation space, such that the water supply tank is separably installed in the tank installation space and is connected to the steam unit, for supplying water to the steam unit, a drainage tank disposed in the tank installation space, such that the drainage tank is separably installed in the tank installation space, for storing condensed water generated in at least one selected from between the treatment chamber and the heat pump unit, a water supply level sensor disposed in the water supply tank configured to directly sense a level of water stored in the water supply tank, and a drainage level sensor disposed in the drainage tank configured to directly sense a level of water stored in the drainage tank. In the clothes treatment apparatus, the level of water stored in the water supply tank or the drainage tank is directly sensed. Consequently, it is possible to sense the level of water stored in the water supply tank or the drainage tank without delay and to secure a sufficient space to store to be supplied during one cycle or condensed water, thereby improving the reliability of operation of the clothes treatment apparatus.

## Description

The present invention relates to a clothes treatment apparatus.

Clothes treatment apparatuses are apparatuses that treat clothes, e.g. wash and dry clothes and smooth wrinkles in clothes, at home or at laundromats.

Clothes treatment apparatuses may be classified into a washer for washing clothes, a dryer for drying clothes, a washer/dryer having both a washing function and a drying function, a refresher for refreshing clothes, and a steamer for removing unnecessary wrinkles in clothes.

The refresher is an apparatus that keeps clothes comfortable and fresh. The refresher functions to dry clothes, to supply fragrance to clothes, to prevent the occurrence of static electricity in clothes, or to remove wrinkles from clothes.

The steamer is an apparatus that simply supplies steam to clothes in order to remove wrinkles from the clothes. Unlike a general iron, the steamer removes wrinkles from the clothes without directly applying heat to the clothes.

A clothes treatment apparatus having both functions of a refresher and a steamer may remove wrinkles from clothes received in the clothes treatment apparatus, and may additionally deodorize the clothes, using steam and hot air.

An example of such a conventional treatment apparatus is disclosed in Korean Patent Application Publication No. 10-2014-0016093.

Therefore, the present invention has been made in view of the above problems, and it is an object of the present invention to provide a clothes treatment apparatus that is capable of directly sensing the level of water stored in a tank.

It is another object of the present invention to provide a clothes treatment apparatus that is capable of enabling a user to immediately check fir the deficiency of water during the operation of the clothes treatment apparatus.

The objects are solved by the independent claim. The dependent claims relate to further aspects of the invention.

In accordance with an aspect of the present invention, the above and other objects can be accomplished by the provision of a clothes treatment apparatus including a cabinet partitioned into a treatment chamber for allowing clothes to be hung therein, a cycle chamber for allowing machinery to be installed therein, and a tank installation space for allowing a removable tank to be installed therein, a door for opening and closing the cabinet, a steam unit disposed in the cycle chamber for supplying steam into the treatment chamber, a heat pump unit disposed in the cycle chamber for circulating air in the treatment chamber and conditioning the air that is circulated, a water supply tank disposed in the tank installation space, such that the water supply tank is separably installed in the tank installation space and is connected to the steam unit, for supplying water to the steam unit, a drainage tank disposed in the tank installation space, such that the drainage tank is separably installed in the tank installation space, for storing condensed water generated in at least one selected from between the treatment chamber and the heat pump unit, a water supply level sensor disposed in the water supply tank configured to directly sense the level of water stored in the water supply tank, and a drainage level sensor disposed in the drainage tank configured to directly sense the level of water stored in the drainage tank.

The clothes treatment apparatus may further include a partition plate for partitioning the interior of the cabinet into upper and lower interior parts such that the treatment chamber and the cycle chamber are partitioned from each other, and a tank module frame for partitioning the interior of the cabinet below the partition plate into front and rear interior parts such that the cycle chamber and the tank installation space are partitioned from each other.

The tank installation space may be formed so as to face the door.

The water supply tank and the drainage tank may be arranged parallel to each other in rightward and leftward directions.

The clothes treatment apparatus may further include a tank support bar disposed between the tank installation space and the door, wherein at least one selected from between the water supply tank and the drainage tank may be disposed so as to be placed on the tank support bar.

The at least one selected from between the water supply tank and the drainage tank may be provided with a tank support end, the tank support end interfering with the tank support bar, the tank support end being concavely recessed.

The water supply tank or the drainage tank, placed on the tank support bar, may form a continuous surface with the tank support bar.

The upper side of at least one selected from between the water supply tank and the drainage tank may be round such that, when the at least one selected from between the water supply tank and the drainage tank is separated, interference between the at least one selected from between the water supply tank and the drainage tank and the partition plate is minimized.

At least one selected from between the water supply tank and the drainage tank may be provided with a grip, the grip being formed at the at least one selected from between the water supply tank and the drainage tank such that the grip is concave from the front to the rear thereof.

The water supply level sensor may include a float case fixed in the water supply tank, a float installed in the float case such that the float is movable upward and downward in the float case by buoyancy, and a sensor installed at the cabinet configured to sense a magnetic force of the float.

The sensor may be installed in any one selected from between the cycle chamber and the tank installation space.

The float case of the water supply level sensor may be disposed at a position at which water remains in an amount that is sufficient to be supplied to the steam unit during one cycle.

The drainage level sensor may include a float case fixed in the drainage tank, a float installed in the float case such that the float is movable upward and downward in the float case by buoyancy, and a sensor installed at the cabinet configured to sense a magnetic force of the float.

The sensor may be installed in any one selected from between the cycle chamber and the tank installation space.

The float case of the drainage level sensor may be disposed at a position at which capacity remains to store an amount of condensed water generated during the cycle in the at least one selected from between the treatment chamber and the heat pump unit.

The water supply tank or the drainage tank may include a tank body that is open at the front thereof, the upper side of the surface of the tank body that is inserted into the tank installation space being round, a tank cover coupled to the front of the tank body, the tank cover being provided with a grip, the grip being concavely formed inward, and a check valve installed in the tank body for opening and closing a flow channel extending from the tank body to the outside.

The clothes treatment apparatus may further include a tank support bar disposed between the tank installation space and the door, wherein at least one selected from between the water supply tank and the drainage tank may be disposed so as to be placed on the tank support bar, and the at least one selected from between the water supply tank and the drainage tank may be provided with a tank support end, the tank support end being placed on the upper side of the tank support bar such that the tank support end interferes with the tank support bar, the tank support end being recessed rearward.

The check valve installed in the water supply tank may be disposed at the lower side of the tank body, and may be connected to the steam unit to supply water to the steam unit.

The clothes treatment apparatus may further include a water hole formed at the upper side of the tank body and a water hole cover for opening and closing the water hole.

The water supply tank or the drainage tank may further include a float installation part formed in the tank body, the tank body and the tank cover may be manufactured by insert injection molding using die slide injection (DSI), and the water supply level sensor or the drainage level sensor may be installed in the float installation part by insert injection molding using DSI.

### BRIEF DESCRIPTION OF THE DRAWINGS

The embodiments will be described in detail with reference to the following drawings in which like reference numerals refer to like elements wherein:
FIG. 1 is a perspective view of a clothes treatment apparatus according to a first embodiment of the present invention;
FIG. 2 is an exploded perspective view of a cycle assembly according to a first embodiment of the present invention;
FIG. 3 is a perspective view of the cycle assembly according to the first embodiment of the present invention;
FIG. 4 is an exploded perspective view of a water supply tank shown in FIG. 1;
FIG. 5 is a partially exploded perspective view of the water supply tank shown in FIG. 1;
FIG. 6 is a sectional perspective view of a check assembly shown in FIG. 5;
FIG. 7 is a side sectional view of the water supply tank shown in FIG. 1;
FIG. 8 is a perspective view of a drainage tank shown in FIG. 1;
FIG. 9 is a partially exploded perspective view of the drainage tank shown in FIG. 1;
FIG. 10 is a side sectional view of the drainage tank shown in FIG. 1; and
FIG. 11 is a block diagram of the clothes treatment apparatus shown in FIG. 1.

### DETAILED DESCRIPTION OF THE PREFERRED EMBODIMENTS

The present invention will be described in detail with reference to the accompanying drawings.

In the following description of the present invention, a detailed description of known functions or configurations incorporated herein will be omitted when it may make the subject matter of the present invention rather unclear. The same terms may be denoted by different reference numerals if the terms indicate different parts.

The terms used in the following description are terms defined taking into consideration the functions obtained in accordance with the present invention. The definitions of these terms should be determined based on the whole content of this specification because they may be changed in accordance with the intentions of users, such as experimenters and measurers, or usual practices.

In this specification, the terms "first," "second," etc. are used to describe various elements. However, the elements are not limited by the terms. The terms are used only to distinguish one element from another element. For example, a first element may be named a second element, and a second element may be named a first element, without departing from the scope of right of the present invention. It will be understood that the term "and/or" refers to one or more possible combinations of specified relevant items and includes such combinations.

The terms used in this specification are provided only to explain specific embodiments, but are not intended to restrict the present invention. A singular representation may include a plural representation unless it represents a definitely different meaning from the context.

Unless otherwise defined, all terms, including technical and scientific terms, used in this specification have the same meaning as commonly understood by a person having ordinary skill in the art to which the present invention pertains. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

In addition, the terms "comprises" and "includes" described herein should be interpreted not to exclude other elements but to further include such other elements since the corresponding elements may be inherent unless mentioned otherwise.

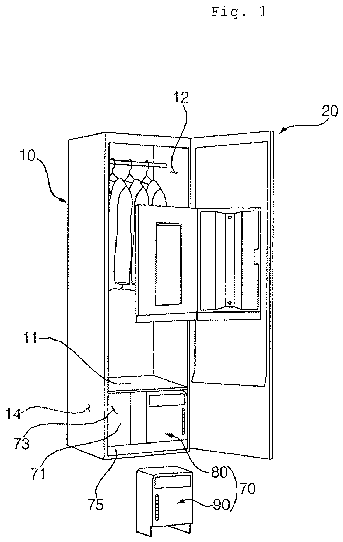

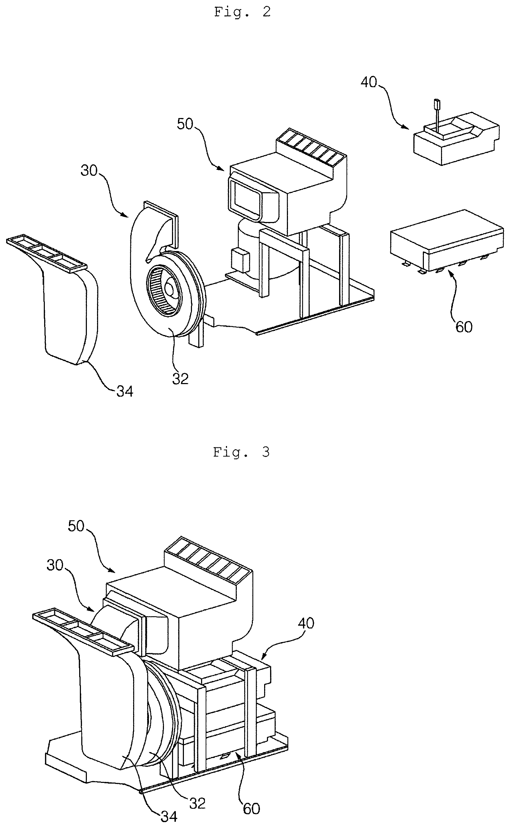

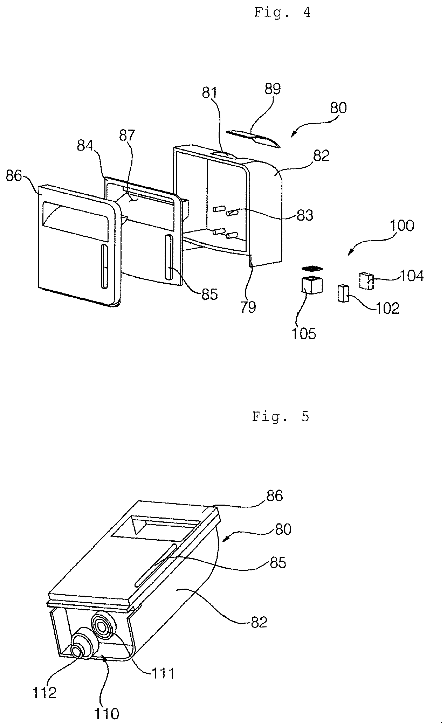

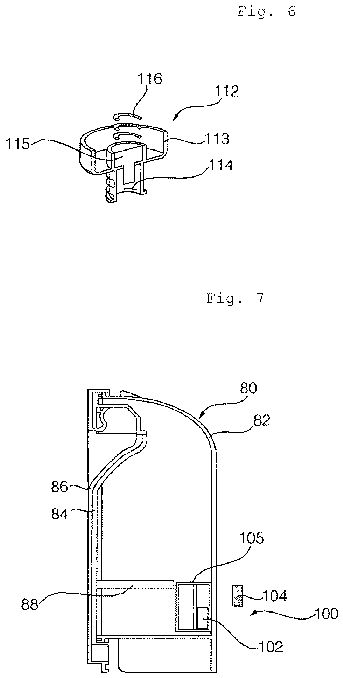

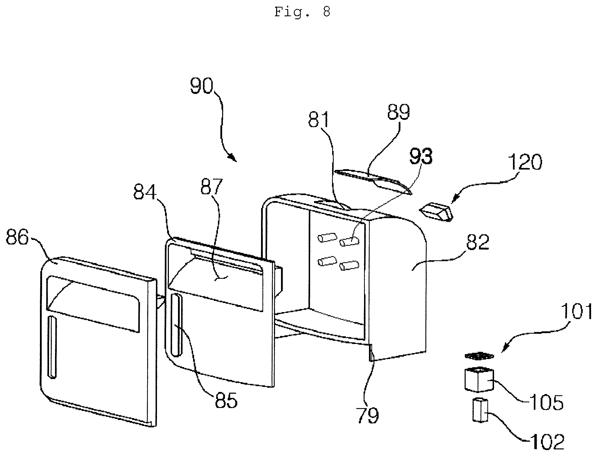

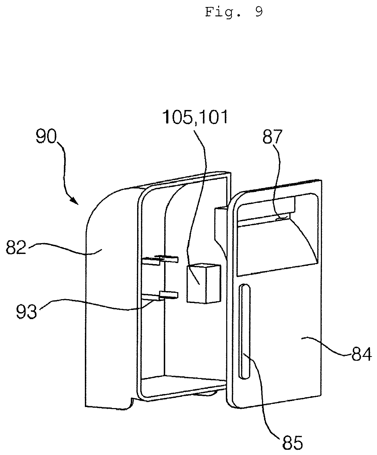

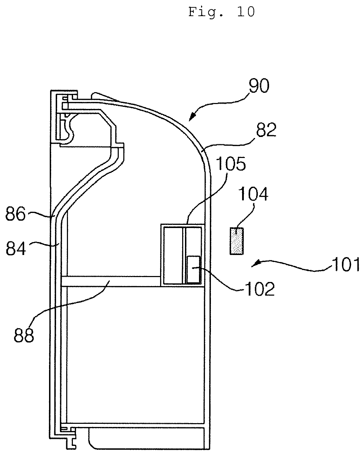

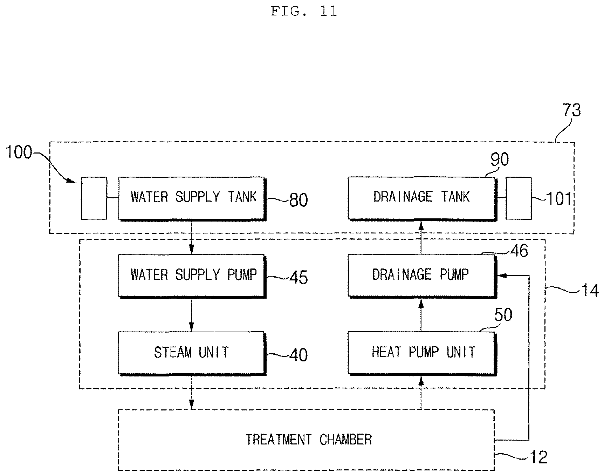

FIG. 1 is a perspective view of a clothes treatment apparatus according to a first embodiment of the present invention, FIG. 2 is an exploded perspective view of a cycle assembly according to a first embodiment of the present invention, FIG. 3 is a perspective view of the cycle assembly according to the first embodiment of the present invention, FIG. 4 is an exploded perspective view of a water supply tank shown in FIG. 1, FIG. 5 is a partially exploded perspective view of the water supply tank shown in FIG. 1, FIG. 6 is a sectional perspective view of a check assembly shown in FIG. 5, FIG. 7 is a side sectional view of the water supply tank shown in FIG. 1, FIG. 8 is a perspective view of a drainage tank shown in FIG. 1, FIG. 9 is a partially exploded perspective view of the drainage tank shown in FIG. 1, FIG. 10 is a side sectional view of the drainage tank shown in FIG. 1, and FIG. 11 is a block diagram of the clothes treatment apparatus shown in FIG. 1.

The clothes treatment apparatus according to this embodiment includes a cabinet 10 and a door 20 configured to open and close the front of the cabinet 10.

The interior of the cabinet 10 is partitioned into upper and lower interior parts by a partition plate 11. A treatment chamber 12, in which clothes are hung, is defined in the interior of the cabinet 10 above the partition plate 11. A cycle chamber 14, in which machinery is installed, is defined in the interior of the cabinet 10 below the partition plate 11.

Clothes are hung in the treatment chamber 12. In the treatment chamber 12, wrinkles in the clothes are smoothed, or the clothes are deodorized, by the circulation of steam or air.

A blowing unit 30 for circulating air in the treatment chamber 12, a steam unit 40 for supplying steam into the treatment chamber 12, a heat pump unit 50 for conditioning air in the treatment chamber 12, and a control unit 60 for controlling the respective units 30, 40, and 50 are installed in the cycle chamber 14.

In this embodiment, an assembly of machinery, including the blowing unit 30, the steam unit 40, the heat pump unit 50, and the control unit 60, which are required to perform respective cycles of the clothes treatment apparatus, is defined as a cycle assembly.

The blowing unit 30 includes a blowing fan 32 and an inlet duct 34.

The inlet duct 34 is installed at the suction side of the blowing fan 32 to guide air in the treatment chamber 12 to the blowing fan 32.

The blowing fan 32 is rotated to blow air. The blowing fan 32 suctions air from the treatment chamber 12, and discharges the suctioned air to the heat pump unit 50.

When the steam unit 40 is powered on, heat is generated from the steam unit 40. The steam unit 40 converts water supplied from a water supply tank 80, which will be described hereinafter, into steam. The generated steam is discharged into the treatment chamber 12.

In this embodiment, a flow channel is defined such that the steam flows into the treatment chamber 12 via the heat pump unit 50.

The heat pump unit 50 constitutes a heat pump cycle including a compressor, a condenser, an evaporator, and an expansion valve. Based on the operation mode of the heat pump unit 50, cooled air or heated air may be discharged into the treatment chamber 12.

In particular, the heat pump unit 50 may dehumidify air supplied from the blowing unit 30.

A tank module 70 for storing water is installed in front of the cycle chamber 14. The tank module 70 includes a water supply tank 80 for supplying water to the steam unit 40 and a drainage tank 90 for gathering and storing condensed water that is generated in the treatment chamber 12.

Water from the water supply tank 80 flows to the steam unit 40 via a water supply pump 45.

Water that is condensed in the treatment chamber 12, flows to the lower side of the treatment chamber 12 due to gravity, and is then pumped to the drainage tank 90 by a drainage pump 46. Water that is condensed in the heat pump unit 50 also flows to the drainage tank 90 via the drainage pump 46.

The water supply pump 45 or the drainage pump 46 is controlled by the control unit 60.

In this embodiment, a tank module frame 71 is installed in front of the inlet duct 34.

A tank installation space 73 is defined between the tank module frame 71 and the door 20. The tank module frame 71 is coupled to the partition plate 11 to isolate the cycle chamber 14 from the outside.

A tank support bar 75, which interferes with at least one selected from between the water supply tank 80 and the drainage tank 90, is installed in front of the tank installation space 73.

The tank support bar 75 prevents the water supply tank 80 or the drainage tank 90 from being unintentionally separated from the tank installation space 73. The tank support bar 75 supports the front of the water supply tank 80 and the front of the drainage tank 90.

When the door 20 is opened and closed, therefore, the water supply tank 80 and the drainage tank 90 are prevented from being separated from the tank installation space 73.

In this embodiment, the lower end of the water supply tank 80 is placed on the upper end of the tank support bar 75, and the lower end of the drainage tank 90 is placed on the upper end of the tank support bar 75.

A tank support end 79, which interferes with the tank support bar 75, is formed on at least one selected from between the water supply tank 80 and the drainage tank 90.

The tank support end 79 is concavely recessed.

The front of the tank support bar 75 and the front of the water supply tank 80 may form a continuous surface due to the tank support end 79. In addition, the front of the tank support bar 75 and the front of the drainage tank 90 may form a continuous surface due to the tank support end 79

The water supply tank 80 and the drainage tank 90 are disposed in the tank installation space 73 such that the water supply tank 80 and the drainage tank 90 are arranged parallel to each other in rightward and leftward directions.

When the door 20 is opened, the water supply tank 80 and the drainage tank 90 are exposed to a user.

The water supply tank 80 and the drainage tank 90 may be withdrawn by the user.

The water supply tank 80 and the drainage tank 90 may be separated from the tank module frame 71. The water supply tank 80 and the drainage tank 90 may be separably mounted in the tank installation space 73.

The water supply tank 80 is connected to the steam unit 40 to supply water to the steam unit 40. The drainage tank 90 is connected to the treatment chamber 12 to store water discharged from the treatment chamber 12 or the heat pump unit 50.

The water supply tank 80 includes a tank body 82, which is open at the front thereof, a tank cover 84 coupled to the front of the tank body 82, a decorative cover 86 coupled to the tank cover 84, a water supply check valve 110 installed in the tank body 82 for opening and closing a flow channel connected with the steam unit 40, and a water supply level sensor 100 for sensing the level of water stored in the tank body 82.

The front of the tank body 82 is open. The water supply level sensor 100 is disposed in the tank body 82.

The upper end of the tank body 82 is round at the rear side thereof.

When the tank body 82 is separated, interference between the tank body 82 and the partition plate 11 is minimized.

The user may easily pull and withdraw the water tank 80, which is disposed at the lower side of the clothes treatment apparatus, due to the round shape of the tank body 82.

In this embodiment, the water supply level sensor 100 includes a float 102 installed in the tank body 82 such that the float 102 can move upward and downward based on the level of water stored in the tank body 82, a float cabinet 105 installed in the tank body 82 in a state in which the float 102 is disposed in the float cabinet 105, and a sensor 104 installed at the tank module frame 71 to sense the float 102.

The float 102 has a magnet. The sensor 104 senses the magnetic force of the magnet.

The sensor 104 may be installed at the front or rear of the tank module frame 71.

The sensor 104 may be installed through the tank module frame 71.

Consequently, the sensor 104 may be located in any one selected from among the cycle chamber 14, the tank installation space 73, and the tank module frame 71.

The float 102, which is installed in the water supply tank 80, is flush with the sensor 104. When the level of water stored in the water supply tank 80 is lowered, the float 102 moves lower than the sensor 104. When the sensor 104 fails to sense the float 102, therefore, the control unit 60 outputs a water deficiency signal. Even when the water deficiency signal is output, it is possible to supply a sufficient amount of steam during a cycle that is currently being performed.

Since the sensor 104 constantly senses the float 102, the control unit 60 may determine whether the water supply tank 80 is mounted.

For example, when the water supply tank 80 is not mounted, or when water is deficient, the control unit 60 outputs a water deficiency signal.

When the user manipulates the clothes treatment apparatus in a state in which the water deficiency signal is output, therefore, the control unit 60 performs control such that the clothes treatment apparatus is not operated and outputs a water deficiency signal. At this time, the user may check the water supply tank 80.

A float installation part 83, at which the float 102 is installed, is formed at the inside of the tank body 82. The float cabinet 105 is installed at the float installation part 83. The float 102 may move upward and downward along the float cabinet 105 by buoyancy.

In this embodiment, the float 102 is installed at the minimum level of water stored in the water supply tank 80, at which it is possible to supply an amount of steam corresponding to one cycle. Even when the sensor 104 fails to sense the float 102, and therefore the control unit 60 outputs a water deficiency signal, it is possible to supply an amount of steam corresponding to at least one cycle.

That is, even when a water deficiency signal is sensed during the supply of steam, it is possible to supply a sufficient amount of steam until a cycle that is currently being performed is completed.

The float cabinet 105, in which the float 102 is mounted, is manufactured by insert injection molding at the time of die slide injection (DSI) of the tank cover 84 and the tank body 82.

Die slide injection (DSI) is a molding technology that has been developed for blow molding or molding of thin products. DSI conveys various advantages in that no post-processing, such as adhesion or assembly, is necessary after injection molding, it is possible to adjust the thickness of a wall more easily than when blow molding or gas molding, it is possible to provide an excellent surface shape or high dimensional accuracy, and it is possible to perform DSI more easily than double injection or blow molding. The manufacturing of products using DSI is ordinarily known in the art to which the present invention pertains, and therefore a detailed description thereof will be omitted.

The tank body 82 and the tank cover 84 are manufactured by insert injection molding using DSI. During the manufacturing of the tank body 82 and the tank cover 84, the float cabinet 105 is installed in the tank body 82 and the tank cover 84 by insert injection molding. During the manufacturing of the tank body 82 and the tank cover 84, the edge of the tank cover 84 is integrally coupled to the edge of the tank body 82.

The tank cover 84 has a window 85, through which the user may check the level of water in the tank body 82. In addition, a grip 87, into which the user may insert his/her hand in order to hold the tank cover 84, is concavely formed at the tank cover 84.

The grip 87 is formed at the tank cover 84 such that the grip 87 is concave from the front to the rear thereof.

A sensor fixing part 88 is formed at the inside of the tank cover 84. The sensor fixing part 88 protrudes from the inside of the tank cover 84. When the tank cover 84 and the tank body 82 are coupled to each other, the sensor fixing part 88 comes into tight contact with the float cabinet 105.

Since the sensor fixing part 88 tightly contacts the float cabinet 105, the float cabinet 105 is prevented from being separated from the float installation part 83.

The sensor fixing part 88 may be integrally formed with the tank cover 84.

The decorative cover 86 is formed to have a shape that is capable of covering the front of the tank cover 84. In addition, the decorative cover 86 is formed to have a shape corresponding to the shape of the tank cover 84.

A water hole 81 is formed at the upper side of the tank body 82. In addition, a water hole cover 89 for opening and closing the water hole 81 is disposed at the upper side of the tank body 82.

The water hole cover 89 is made of a flexible material exhibiting high elasticity. One end of the water hole cover 89 is fixed to the tank body 82, and the other end of the water hole cover 89 may be bent in order to open and close the water hole 81.

The water supply check valve 110 includes a check valve hole 111 formed at the lower side of the tank body 82 and a check assembly 112 coupled to the check valve hole 111 for regulating the water in the tank body 82.

The check assembly 112 includes a check housing 113 coupled into the check valve hole 111, the check housing 113 having a check flow channel 114, through which water flows into the check housing 113, a valve 115 disposed in the check housing 113 for opening and closing the check flow channel 114, and a check elastic member 116 disposed between the valve 115 and the tank body 82 for applying elastic force to the valve 115.

The small-diameter side of the valve 115 protrudes downward. When the valve 115 is placed on the tank module frame 71, the valve 115 may be pushed by the tank module frame 71, and may thus move upward. At this time, the check flow channel 114 is opened as the result of the movement of the valve 115. When the water supply tank 80 is separated from the tank module frame 71, the check flow channel 114 is closed by the elastic force of the check elastic member 116.

The drainage tank 90 is identical in function to the water supply tank 80. The drainage tank 90 is disposed alongside the water supply tank 80.

In the drainage tank 90, a drainage check valve 120 is installed at the rear side thereof, not at the lower side thereof, unlike the water supply tank 80.

The water supply tank 80 receives water through the water hole 81, and discharges water through the water supply check valve 110. The drainage tank 90 may receive condensed water through the drainage check valve 120, and may discharge condensed water through the water hole 81.

That is, the drainage check valve 120 of the drainage tank 90 may be disposed in a channel for receiving condensed water, not for discharging condensed water.

Unlike this embodiment, condensed water may fall into the drainage tank 90 through the water hole 81. In addition, condensed water may be automatically discharged through the drainage check valve 120.

Water that is condensed in the treatment chamber 12 and water that is condensed in the heat pump unit 50 are stored in the drainage tank 90.

A float installation part 93, at which the float cabinet 105 is installed, is formed in the drainage tank 90.

The float installation part 93 may be located at a height in the drainage tank 90 at which overflow does not occur even when an amount of condensed water that is generated during one cycle is stored therein.

That is, the float installation part 93 is located at a height in the drainage tank 90 at which overflow does not occur even when an amount of condensed water that is generated during one cycle is stored in the drainage tank 90.

When a drainage level sensor 101 of the drainage tank 90 senses a signal during the operation of the clothes treatment apparatus, therefore, the water in the drainage tank 90 does not overflow due to the condensed water that is additionally stored in the drainage tank 90.

The drainage level sensor 101 of the drainage tank 90 is located higher than the water supply level sensor 100 in the water supply tank 80.

The drainage level sensor 101 of the drainage tank 90 is identical in construction to the water supply level sensor 100 of the water supply tank 80. However, the drainage level sensor 101 of the drainage tank 90 is operated differently from the water supply level sensor 100 of the water supply tank 80.

For example, the sensor 104 of the drainage tank 90 does not sense the float 102 in a normal state. When the level of condensed water rises, the sensor 104 of the drainage tank 90 senses the float 102, which has been raised by buoyancy.

When the sensor 104 of the drainage tank 90 senses the float 102, the control unit 60 outputs a water drainage signal. When the water drainage signal is output, however, the overflow of condensed water does not occur during a cycle that is currently being performed.

As is apparent from the above description, the clothes treatment apparatus according to the present invention has the following effects.

It is possible to directly sense the amount of water stored in the water supply tank instead of estimating the amount of water stored in the water supply tank.

It is possible to sense the level of water stored in the water supply tank without delay.

It is possible to directly sense the level of water stored in the drainage tank without delay, thereby preventing water from overflowing the drainage tank.

In the clothes treatment apparatus according to the present invention, the water supply level sensor is installed at the level of water that is required to generate enough steam for at least one cycle. Consequently, it is possible to prevent the supply of water from being interrupted while steam is being generated.

In the clothes treatment apparatus according to the present invention, the drainage level sensor is installed at the level of water at which it is possible to store all of the water that is condensed during at least one cycle. Consequently, it is possible to prevent the condensed water from overflowing the drainage tank, or it is not necessary to drain the condensed water from the drainage tank, during the operation of the clothes treatment apparatus.

It will be apparent that, although the embodiments of the present invention have been described above with reference to the accompanying drawings, the present invention is not limited to the above-described specific embodiments, and therefore various modifications and variations can be made by those skilled in the art without departing from the gist of the appended claims. Thus, it is intended that the modifications and variations should not be understood independently of the technical prospect of the present invention. The above embodiments are therefore to be construed in all aspects as illustrative and not restrictive.

The invention further relates to the following items:
1. A clothes treatment apparatus comprising:
   a cabinet (10) partitioned into a treatment chamber (12) for allowing clothes to be hung therein, a cycle chamber (14) for allowing machinery to be installed therein, and a tank installation space (73) configured to allow a removable tank to be installed therein;
   a door (20) for opening and closing the cabinet (10);
   a steam unit (40) disposed in the cycle chamber (14) for supplying steam into the treatment chamber (12);
   a heat pump unit (50) disposed in the cycle chamber (14) for circulating air in the treatment chamber (12) and conditioning the air that is circulated;
   a water supply tank (80) disposed in the tank installation space (73), such that the water supply tank (80) is separably installed in the tank installation space (73) and is connected to the steam unit (40), configured to supply water to the steam unit (40);
   a drainage tank (90) disposed in the tank installation space (73), such that the drainage tank (90) is separably installed in the tank installation space (73), configured to store condensed water generated in at least one selected from between the treatment chamber (12) and the heat pump unit (50);
   a water supply level sensor (100) disposed in the water supply tank (80) configured to directly sense a level of water stored in the water supply tank (80); and
   a drainage level sensor (101) disposed in the drainage tank (90) configured to directly sense a level of water stored in the drainage tank (90).
2. The clothes treatment apparatus according to item 1, further comprising:
   a partition plate (11) for partitioning an interior of the cabinet (10) into upper and lower interior parts such that the treatment chamber (12) and the cycle chamber (14) are partitioned from each other; and
   a tank module frame (71) for partitioning the interior of the cabinet (10) below the partition plate (11) into front and rear interior parts such that the cycle chamber (14) and the tank installation space (73) are partitioned from each other.
3. The clothes treatment apparatus according to items 1 or 2, wherein the tank installation space (73) is formed so as to face the door (20), and/or
   wherein the water supply tank (80) and the drainage tank (90) are arranged parallel to each other in rightward and leftward directions.
4. The clothes treatment apparatus according to any one of items 1 to 3, further comprising a tank support bar (75) disposed between the tank installation space (73) and the door (20), wherein at least one selected from between the water supply tank (80) and the drainage tank (90) is disposed so as to be placed on the tank support bar (75).
5. The clothes treatment apparatus according to item 4, wherein the at least one selected from between the water supply tank (80) and the drainage tank (90) is provided with a tank support end (79), the tank support end (79) interfering with the tank support bar (75), the tank support end (79) being concavely recessed, and/or
   wherein the water supply tank (80) or the drainage tank (90), placed on the tank support bar (75), forms a continuous surface with the tank support bar (75).
6. The clothes treatment apparatus according to any one of items 1 to 5, wherein an upper end of at least one selected from between the water supply tank (80) and the drainage tank (90) is round such that, when the at least one selected from between the water supply tank (80) and the drainage tank (90) is separated, interference between the at least one selected from between the water supply tank (80) and the drainage tank (90) and the partition plate (11) is minimized.
7. The clothes treatment apparatus according to any one of items 1 to 6, wherein at least one selected from between the water supply tank (80) and the drainage tank (90) is provided with a grip (87), the grip (87) being formed at the at least one selected from between the water supply tank (80) and the drainage tank (90) such that the grip (87) is concave from a front to a rear thereof.
8. The clothes treatment apparatus according to any one of items 1 to 7, wherein the water supply level sensor (100) comprises:
   a float case(105) fixed in the water supply tank (80);
   a float (102) installed in the float case (105) such that the float (102) is movable upward and downward in the float case (105) by buoyancy; and
   a sensor (104) installed at the cabinet (10) and configured for sensing a magnetic force of the float (102).
9. The clothes treatment apparatus according to item 8, wherein the sensor (104) is installed in any one selected from between the cycle chamber (14) and the tank installation space (73), and/or
   wherein the float case (105) of the water supply level sensor (100) is disposed at a position at which water remains in an amount that is sufficient to be supplied to the steam unit during one cycle.
10. The clothes treatment apparatus according to any one of items 1 to 9, wherein the drainage level sensor (101) comprises:
   a float case (105) fixed in the drainage tank (90);
   a float (102) installed in the float case (105) such that the float (102) is movable upward and downward in the float case (105) by buoyancy; and
   a sensor (104) installed at the cabinet (10) configured to sene a magnetic force of the float (102).
11. The clothes treatment apparatus according to item 10, wherein the sensor (104) is installed in any one selected from between the cycle chamber (14) and the tank installation space (73), and/or
   wherein the float case (105) of the drainage level sensor (101) is disposed at a position at which capacity remains to store an amount of condensed water generated during the cycle in the at least one selected from between the treatment chamber (12) and the heat pump unit (50).
12. The clothes treatment apparatus according to any one of items 1 to 11, wherein the water supply tank (80) or the drainage tank (90) comprises:
   a tank body (82) open at a front thereof, an upper end of a surface of the tank body (82) that is inserted into the tank installation space (73) and the upper end of a surface of the tank body (82) being round;
   a tank cover (84) coupled to the front of the tank body (82), the tank cover (84) being provided with a grip (87), the grip (87) being concavely formed inward; and
   a check valve (110) installed in the tank body (82) for opening and closing a flow channel extending from the tank body (82) to an outside.
13. The clothes treatment apparatus according to item 12, further comprising a tank support bar (75) disposed between the tank installation space (73) and the door (20), wherein
   at least one selected from between the water supply tank (80) and the drainage tank (90) is disposed so as to be placed on the tank support bar (75), and
   the at least one selected from between the water supply tank (80) and the drainage tank (90) is provided with a tank support end (79), the tank support end (79) being placed on an upper side of the tank support bar (75) such that the tank support end (79) interferes with the tank support bar (75), the tank support end (79) being recessed rearward, and/or
   wherein the check valve (110) installed in the water supply tank (80) is disposed at a lower side of the tank body (82), and is connected to the steam unit (40) to supply water to the steam unit (40).
14. The clothes treatment apparatus according to items 12 or 13, further comprising:
   a water hole (81) formed at an upper side of the tank body; and
   a water hole cover (89) for opening and closing the water hole (81).
15. The clothes treatment apparatus according to any one of items 12 to 14, wherein the water supply tank (80) or the drainage tank (90) further comprises a float installation part (83) formed in the tank body (82), the tank body (82) and the tank cover (84) are manufactured by insert injection molding using die slide injection (DSI), and the water supply level sensor (100) or the drainage level sensor (101) is installed in the float installation part (83) by insert injection molding using DSI.

## Claims

1. A clothes treatment apparatus comprising:
a cabinet (10) that defines an opening at a front side of the cabinet (10);
a door (20) coupled to the cabinet (10) and configured to open and close the opening of the cabinet (10);
a treatment chamber (12) located inside the cabinet (10) and configured to receive clothes through the opening of the cabinet (10);
a cycle chamber (14) located below the treatment chamber (12) ;
a steam unit (12) located in the cycle chamber (14) and configured to supply steam to the treatment chamber (12);
a water tank (80, 90) that defines a water storage space for storing water;
a tank module frame (71) located below the treatment chamber (12) and forward of the cycle chamber (14), the tank module frame (71) defining a tank installation space (73) configured to detachably receive the water tank (80, 90); and
a bar (75) provided at a lower front side of the tank module frame (71),
wherein a front surface of the water tank (80, 90) is exposed to an outside based on the door (20) being open,
wherein the bar (75) is configured to engage with a lower portion of a front part of the water tank (80, 90) to thereby restrict movement of the water tank (80, 90) within the tank module frame (71).

2. The clothes treatment apparatus according to claim 1, wherein the bar (75) is extended vertically from a front side of the tank module frame (71) toward the treatment chamber (12).

3. The clothes treatment apparatus according to claim 1 or 2, wherein a front surface of the bar (75) and the front surface of the water tank (80, 90) form a coplanar surface based on the water tank (80, 90) being received in the tank module frame (71).

4. The clothes treatment apparatus according to any one of claims 1 to 3, wherein the bar (75) is located between the tank installation space (73) and the door (20).

5. The clothes treatment apparatus according to any one of claims 1 to 4, wherein the water tank (80, 90) further comprises:
a grip (87) provided at an upper portion of a front side of the water tank (80, 90), the grip (87) being curved concavely toward the cycle chamber (14).

6. The clothes treatment apparatus according to claim 5, wherein the water tank (80, 90) is configured to be detached from the tank module frame (71) by a rotation of the water tank (80, 90) around the bar (75) based on the grip (87) being pulled by a user.

7. The clothes treatment apparatus according to any one of claims 1 to 6, wherein the water tank (80, 90) comprises:
a tank body (82) defining a front opening and having a bottom side that corresponds to the bottom side of the water storage space; and
a tank cover (84) coupled to the tank body (82) and covering the front opening, the tank cover (84) and the tank body (82) defining the water storage space.

8. The clothes treatment apparatus according to claim 7, wherein the tank cover (84) is integrally coupled to the tank body (82) to thereby prevent leakage.

9. The clothes treatment apparatus according to claim 7 or 8, wherein the tank body (82) comprises:
a first supporting part and a second supporting part that are each vertically elongated from the bottom side of the tank body (82) along a front-rear direction of water tank (80, 90),
wherein the first supporting part is spaced apart from the second supporting part, and the first supporting part and the second supporting part are configured to support the water tank (80, 90) received in the tank installation space (73).

10. The clothes treatment apparatus according to any one of claims 7 to 9, wherein the tank body (82) further comprises:
a rear supporting part vertically elongated from the bottom side of the tank body (82).

11. The clothes treatment apparatus according to any one of claims 7 to 10, wherein the water tank (80, 90) further comprises:
a decorative cover (86) coupled to the tank cover (84) for covering the tank cover (84).

12. The clothes treatment apparatus according to claim 11, wherein the tank cover (84) includes:
a recessed portion provided at an upper portion of a front side of the water tank (80, 90) and concaved toward the cycle chamber (14); and
a recessed opening in communication with a recessed space defined by the recessed portion (87),
wherein the decorative cover (86) defines an insertion in communication with the recessed space through the recessed opening, and
wherein an upper side of an inner surface of the insertion opening is located lower than an upper side of an inner surface of the recessed opening such that the decorative cover (86) covers at least a part of the recessed opening.

13. The clothes treatment apparatus according to claim 12, wherein the decorative cover (86) defines a communication hole (85) through which a water level in the water tank (80, 90) is viewable.

14. The clothes treatment apparatus according to claim 12 or 13, wherein the tank body (82) further includes a a first supporting part and a second supporting part that are each vertically elongated from the bottom side of the tank body (82) along a front-rear direction of the cabinet (10), wherein the first supporting part is spaced apart from the second supporting part, and the first supporting part and the second supporting part are configured to support the water tank (80, 90) received in the tank installation space (73), and
wherein the decorative cover (86) and the tank cover (84) are, based on the water tank (80, 90) being received in the tank installation space (73), spaced apart from the first supporting part and the second supporting part.

15. The clothes treatment apparatus according to claim 14, wherein each of the first supporting part and the second supporting part includes a tank support end located at each front surface of the first supporting part and the second supporting part toward the tank cover (84), and
wherein a predetermined gap is formed between the tank cover (84) and the tank support end.
